**Europäisches Patentamt**

**European Patent Office**

**Office européen des brevets**

(11) Numéro de publication: **0 253 734**
**A1**

## DEMANDE DE BREVET EUROPEEN

(21) Numéro de dépôt: **87401664.5**

(22) Date de dépôt: **15.07.87**

(51) Int. Cl.4: **A 61 N 5/06**

(30) Priorité: **15.07.86 FR 8610286**

(43) Date de publication de la demande:
**20.01.88 Bulletin 88/03**

(84) Etats contractants désignés:
**AT BE CH DE ES GB GR IT LI LU NL SE**

(71) Demandeur: **Société Civile de Recherche "SCR Recherches et Développements"**
**96, rue Damrémont**
**F-75018 Paris (FR)**

(72) Inventeur: **Gasquet, Gilbert**
**2, ruelle du Présbytère Rampillon**
**F-77370 Nangis (FR)**

**Tessier, Bernard**
**18, rue du Grand Prieuré**
**F-75013 Paris (FR)**

(74) Mandataire: **Nony, Michel et al**
**Cabinet NONY & CIE 29, rue Cambacérès**
**F-75008 Paris (FR)**

(54) **Appareil à laser à usage médical.**

(57) L'invention est relative à un appareil à laser à usage médical, comprenant au moins un laser (2) susceptible d'émettre un faisceau de rayonnement infrarouge, et des moyens (4) pour provoquer un balayage dudit faisceau.

Il comprend en outre des moyens (5,10) pour conformer le faisceau de rayonnement infrarouge sous la forme d'un faisceau sensiblement plan, les moyens de balayage sont agencés pour provoquer un balayage alternatif du faisceau dans une direction sensiblement perpendiculaire à son plan, et ledit laser est un laser à émission continue.

Fig. 1

EP 0 253 734 A1

**Description**

Appareil à laser à usage médical.

La présente invention concerne un appareil à laser à usage médical, et plus particulièrement un tel appareil du type comprenant au moins un laser susceptible d'émettre un faisceau de rayonnement infrarouge et des moyens pour provoquer un balayage dudit faisceau.

On connait déjà de nombreux appareils de ce type utilisant un faisceau collimaté. Un balayage de ce faisceau est assuré dans deux directions perpendiculaires de manière à couvrir la surface à traiter. Le balayage d'un tel faisceau présente toutefois l'inconvénient que, quels que soient les moyens utilisés (balayage ligne par ligne ou balayage par "courbe de Lissajou", par exemple), le faisceau décrit une courbe sur la surface à traiter de sorte que certains points de cette surface ne reçoivent pas de rayonnement.

Cet inconvénient est d'ailleurs généralement amplifié par le fait que l'on utilise des lasers fonctionnant par impulsions, de sorte que les courbes précitées elles-mêmes ne sont en fait qu'une succession de points discrets.

La présente invention vise à pallier ces inconvénients en fournissant un appareil à laser tel que chaque point de la surface à traiter reçoit une partie du rayonnement émis.

A cet effet, l'invention a pour objet un appareil à laser à usage médical, comprenant au moins un laser suceptible d'émettre un faisceau de rayonnement infrarouge et des moyens pour provoquer un balayage dudit faisceau, caractérisé par le fait qu'il comprend en outre des moyens pour conformer le faisceau de rayonnement infrarouge sous la forme d'un faisceau sensiblement plan, que les moyens de balayage sont agencés pour provoquer un balayage alternatif du faisceau dans une direction sensiblement perpendiculaire à son plan, et que ledit laser est un laser à émission continue.

Le faisceau laser utilisé étant par conséquent un faisceau plan, son intersection avec la surface à traiter est une ligne dont on provoque le déplacement alternatif dans une direction perpendiculaire à elle-même. Ainsi, la totalité de la surface à traiter est effectivement balayée par le faisceau et reçoit par conséquent une partie du rayonnement infrarouge émis par le laser. Par ailleurs, l'utilisation d'un laser à émission continue contribue également à ce résultat en évitant que le faisceau décrive en fait sur la surface à traiter une succession de lignes parallèles discrètes.

Dans un mode de réalisation particulier de l'invention, ledit laser infrarouge est un laser à semi-conducteur, les moyens de conformation du faisceau comprenant une optique de collimation et une lentille de défocalisation.

On sait en effet que le faisceau issu d'un laser à semi-conducteur présente une grande divergence. Une première possibilité consiste donc à concentrer ce faisceau dans un plan. On a toutefois constaté qu'il était préférable de le collimater pour obtenir un faisceau sensiblement rectiligne, puis de défocaliser ce faisceau dans une direction de manière à obtenir le faisceau plan de l'invention.

Les moyens de balayage peuvent par exemple comprendre un miroir monté sur un vibreur électromagnétique.

Dans un mode de réalisation préféré, l'appareil selon l'invention comprend un deuxième laser susceptible d'émettre un faisceau de rayonnement visible, des moyens pour conformer ce faisceau de rayonnement visible sous la forme d'un faisceau sensiblement plan ayant sensiblement la même divergence que le faisceau de rayonnement infrarouge, et des moyens pour amener les deux faisceaux à être sensiblement confondus.

Le fait d'utiliser un deuxième faisceau laser dans la gamme des rayonnements visibles, confondu avec le faisceau infrarouge, permet en particulier de visualiser ce dernier et par conséquent de délimiter clairement la zone traitée.

Ce deuxième laser peut être un laser Hélium-Néon, de préférence à polarisation aléatoire.

Les moyens de conformation du faisceau de rayonnement visible peuvent comprendre également une lentille de défocalisation.

Avantageusement, les moyens pour amener les deux faisceaux à être sensiblement confondus comprennent une lame dichroïque disposée suivant le plan bisecteur des faisceaux issus des deux lasers, et dont la fréquence de coupure est comprise entre les fréquences des deux lasers.

On décrira maintenant à titre d'exemple non limitatif, un mode de réalisation particulier de l'invention en référence aux dessins schématiques annexés dans lesquels :

    - la figure 1 est un schéma d'ensemble de l'appareil selon l'invention, et

    - la figure 2 est une vue à plus grande échelle illustrant le fonctionnement de l'appareil de la figure 1.

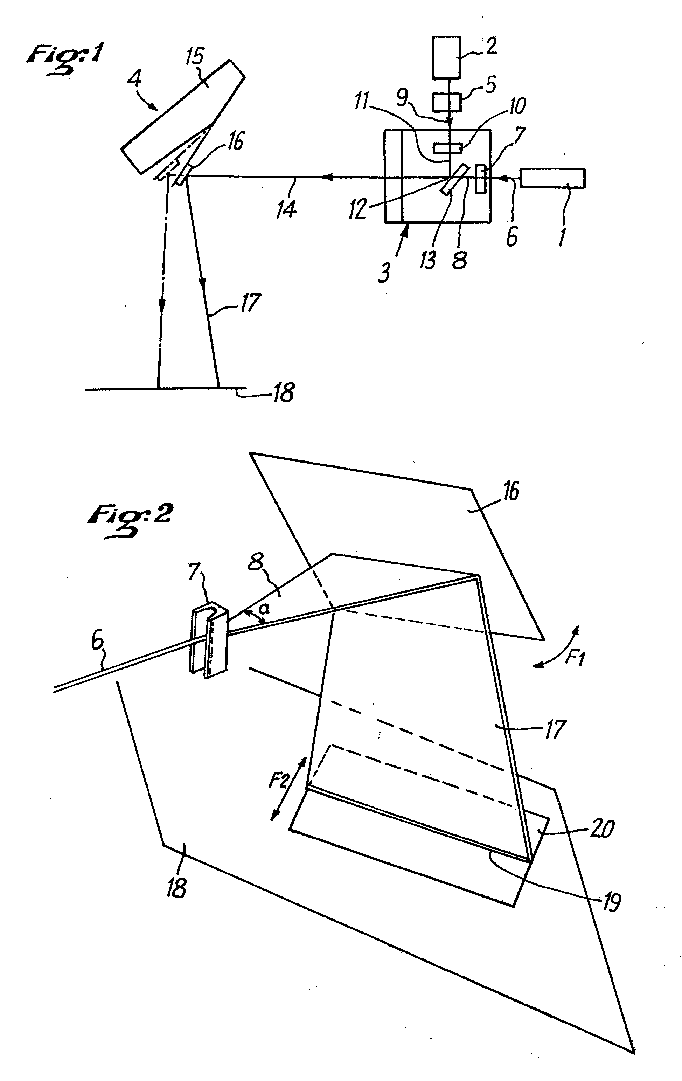

L'appareil de la figure 1 comprend essentiellement deux lasers 1 et 2, un bloc optique 3, et un dispositif de balayage 4.

Dans le cas présent, le laser 1 est un laser Hélium-Néon émettant en continu à 632.8 nm, un faisceau visible de 2,2 mm de diamètre, et le laser 2 est une diode AlGaAs à hétérojonction émettant également en continu dans le proche infrarouge à 820 nm. Le laser 1 fonctionne en mode TEM 22 et le laser 2 en mode TEM00. Le rayonnement issu du laser 1 est à polarisation aléatoire. Une optique de collimation 5 transforme le faisceau fortement divergent issu du laser 2 en un faisceau rectiligne de 5,4 mm de diamètre.

Le faisceau 6 issu du laser Hélium-Néon 1 est dirigé sur une lentille cylindrique divergente 7 qui transforme le faisceau 6 en un faisceau plan divergent 8. Pour une lentille 7 de focale 6,35, la divergence a du faisceau 8 est de 10° 20'.

De même, le faisceau 9 issu de l'optique de collimation 5 est dirigé sur une lentille cylindrique divergente 10 qui transforme le faisceau rectiligne 9 en un faisceau plan divergent 11. Pour une lentille 10 de focale 12,7, la divergence du faisceau 11 est également de 10° 20'.

Le faisceau 6 issu du laser 1, et le faisceau 9 issu du laser 2 par l'intermédiaire de l'optique de collimation 5, sont coplanaires et perpendiculaires l'un à l'autre. Par conséquent, les faisceaux plans divergents 8 et 11 forment un dièdre droit dont l'arête 12 est perpendiculaire au plan de la figure 1.

Une lame dichroïque 13 est placée dans le plan bisecteur de ce dièdre d'arête 12. Cette lame n'est pas représentée à la figure 2, où seul un des faisceaux est représenté pour plus de clarté.

La longueur d'onde de coupure de la lame 13 est de 700 nm, de sorte que le faisceau 8 à 632,8 nm la traverse et que le faisceau 11 à 820 nm est réfléchi.

Il en résulte donc un faisceau bichromatique unique 14, divergent à 10° 20' et situé dans le prolongement du faisceau 8.

Un diaphragme 13' est en outre prévu pour limiter la divergence du faisceau 14.

Le dispositif de balayage 4 se compose d'un vibreur électromagnétique 15 sur lequel est monté un miroir 16, agencé pour vibrer en rotation autour d'un axe parallèle au plan du faisceau 14. Le miroir 16 est disposé de telle sorte qu'il intercepte et réfléchisse ce faisceau 14.

Le faisceau 17 ainsi réfléchi est dirigé sur la surface à traiter 18, sur laquelle il forme une ligne 19 comme représenté à la figure 2.

Lorsque le miroir 16 vibre, comme représenté par la flèche F1, il en résulte que la ligne 19 se déplace selon un mouvement alternatif représenté par la flèche F2, balayant ainsi un rectangle 20.

Les lasers 1 et 2 émettant en continu et le balayage de la zone 20 par le faisceau 17 s'effectuant également en continu, on constate que chaque point de cette zone est atteint par le rayonnement.

Diverses variantes et modifications peuvent bien entendu être apportées à la description qui précède sans sortir pour auant du cadre ni de l'esprit de l'invention.

## Revendications

1. Appareil à laser à usage médical, comprenant au moins un laser (2) susceptible d'émettre un faisceau de rayonnement infrarouge, et des moyens (4) pour provoquer un balayage dudit faisceau, caractérisé par le fait qu'il comprend en outre des moyens (5,10) pour conformer le faisceau de rayonnement infrarouge sous la forme d'un faisceau sensiblement plan, que les moyens de balayage sont agencés pour provoquer un balayage alternatif du faisceau dans une direction sensiblement perpendiculaire à son plan, et que ledit laser est un laser à émission continue.

2. Appareil selon la revendication 1, caractérisé par le fait que ledit laser infrarouge est un laser à semi-conducteur, et que les moyens de conformation du faisceau comprennent une optique de collimation (5) et une lentille de défocalisation (10).

3. Appareil selon l'une quelconque des revendications 1 et 2, caractérisé par le fait que lesdits moyens de balayage comprennent un miroir (16) monté sur un vibreur électromagnétique (15).

4. Appareil selon l'une quelconque des revendications 1 à 3, caractérisé par le fait qu'il comprend un deuxième laser (1), susceptible d'émettre un faisceau de rayonnement visible, des moyens (7) pour conformer ce faisceau de rayonnement visible sous la forme d'un faisceau sensiblement plan ayant sensiblement la même divergence que le faisceau de rayonnement infrarouge, et des moyens (13) pour amener les deux faisceaux à être sensiblement confondus.

5. Appareil selon la revendication 4, caractérisé par le fait que ledit deuxième laser est un laser Hélium-Néon.

6. Appareil selon l'une quelconque des revendications 4 et 5, caractérisé par le fait que ledit faisceau de rayonnement visible est à polarisation aléatoire.

7. Appareil selon l'une quelconque des revendications 4 à 6, caractérisé par le fait que les moyens de conformation du faisceau de rayonnement visible comprennent une lentille de défocalisation.

8. Appareil selon l'une quelconque des revendications 4 à 7, caractérisé par le fait que les moyens pour amener les deux faisceaux à être sensiblement confondus comprennent une lame dichroïque, disposée suivant le plan bisecteur des faisceaux issus des deux lames et dont la fréquence de coupure est comprise entre les fréquences deux deux lasers.

253734

Fig:1

Fig:2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| A | DE-A-1 541 165  (SIEMENS)<br>* Page  16,  ligne 24 - page 17, ligne 18 * | 1,2,7 | A 61 N    5/06 |
| A | DE-A-3 331 586  (MULLER)<br>* Page 7,  ligne  18  -  page  9, ligne 8 * | 4,5,8 | |
| A,P | FR-A-2 577 425  (BOUCHLAGEM)<br>* Page 6, ligne 1 - page 7, ligne 2 * | 3,5 | |
| A | US-A-4 279 472  (STREET)<br>* Colonne 5, lignes 46-64 * | 1,3 | |

---

|  |  |  | DOMAINES TECHNIQUES RECHERCHES (Int. Cl.4) |
|---|---|---|---|
|  |  |  | A 61 N<br>G 02 B |

Le présent rapport de recherche a été établi pour toutes les revendications

| Lieu de la recherche | Date d'achèvement de la recherche | Examinateur |
|---|---|---|
| LA HAYE | 26-10-1987 | LEMERCIER D.L.L. |